# EUROPÄISCHE PATENTANMELDUNG

(11) **EP 1 987 797 A2**
(43) Veröffentlichungstag der Anmeldung: **05.11.2008**
(21) Anmeldenummer: 08400028.0
(22) Anmeldetag: 29.04.2008
(51) Int. Cl.: A61B 19/02

(54) **Trageplatte mit Öffnung zur Aufnahme eines Faches**

(30) Priorität: 03.05.2007 DE 102007021098
(71) Anmelder: Weinmann, 22525 Hamburg (DE)
(72) Erfinder: Die Erfindernennung liegt noch nicht vor
(74) Vertreter: Klickow, Hans-Henning

(57) **Zusammenfassung**

Die Vorrichtung dient zur Notfallversorgung eines Patienten und ist als eine mobile Einheit ausgebildet. Die mobile Einheit besitzt eine Tragstruktur mit einer Rückseite sowie mindestens einer Funktionseinheit. Die Funktionseinheit ist durch einen Träger gehaltert. Der Träger weist mindestens bereichsweise einen Abstand zu einer von der Rückseite aufgespannten Ebene auf.

## Beschreibung

Die Erfindung betrifft eine Vorrichtung zum komfortablen Transport von medizinischen Geräten, beispielsweise in der Notfallmedizin vom Rettungsfahrzeug zum Patienten.

Im Bereich der Notfallbeatmung werden Tragstrukturen zum Transport und zur übersichtlichen Anordnung von Funktionseinheiten/Geräten auf einer Platte verwendet, um medizinische Geräte (bspw. Beatmungsgerät, Absauggerät, Defibrillator, verschiedene Monitor- und Diagnosesystem, etc.) schnell und effizient an den Notfallort zu transportieren. Die Tragstrukturen werden meist an einer Wand in einem Gebäude oder einem Fahrzeug an einer Basisstruktur angebracht. Diese verfügt über mindestens eine Schnittstelle, welche mit einem Teil an der Tragstruktur selbst und mit einem zweiten Teil an der Basisstruktur angebracht ist.

Wird die Tragstruktur in die Basisstruktur eingehängt und eingerastet, so verbindet sich auch die Komponenten der Schnittstelle automatisch. Die Schnittstelle kann die elektrische Kommunikation mit anderen Geräten oder eine Ladung und/ oder eine elektrischen Energieversorgung der auf der Tragstruktur angeordneten Funktionseinheiten sicherstellen.

Bei vielen Rettungsorganisationen und Rettungsdiensten werden Betäubungsmittel (BTM) im Rettungswagen unter Verschluss im Rettungsfahrzeug mitgeführt, da diese Medikamente besonderen Bestimmungen und Betäubungsmittelgesetzen unterliegen, so dass diese nicht frei zugänglich im Rettungsfahrzeug gelagert werden dürfen. Meist befindet sich im Rettungsfahrzeug eine Art Tresor, welcher nur mit einer Nummernkombination oder einem Schlüssel zu öffnen ist.

In vielen Fällen stört jedoch die an der Tragstruktur angebrachte Schnittstelle bei der Anordnung und Anbringung der Geräte bzw. Module, so dass bei der Konfektionierung um die Schnittstelle herum die Geräte angeordnet werden müssen. Die Basisstruktur umfaßt ebenfalls den Gegenpart der Schnittstelle, so dass eine Kompatibilität zu neuen und alten Tragstrukturen gewährleistet sein muss.

Oftmals besteht in einem Behandlungsfall ein Bedarf an diesen Betäubungsmitteln, sei es, um den Patienten zu sedieren oder seine Schmerzen zu senken - insbesondere im Falle einer künstlichen Beatmung des Patienten. Wird die Therapie einer Beatmung gewählt, so müssen die relevanten Betäubungsmittel erst aus dem Fahrzeug beschafft werden, womit wertvolle zeit verloren gehen kann.

Bisher gibt es keine sichere verschlussmöglichkeit für mitzuführende Betäubungsmittel, die der Arzt oder Anwender mit sich führen kann.

Des Weiteren ist es bei Geräten, welche auf einer Tragstruktur angeordnet sind, oftmals schwierig, eine schnelle und einfache Wartung durchzuführen. D.h., dass die Geräte erst von der Tragstruktur gelöst werden müssen, um durch weiteres Lösen eines Gehäuses des betreffenden Gerätes an das Innere des Gerätes zu gelangen.

Ferner ist es oftmals schwierig, Begleitinformationen sowie sonstige Dokumentation über die einzelnen Funktionseinheiten direkt an den Funktionseinheiten aufzubewahren. Vielfach werden diese dann nicht mitgeführt oder finden im Fahrzeug einen Aufbewahrungsort, wo sie bei Störungen und anderen Fragestellungen zu den auf der Tragstruktur angebrachten Funktionseinheiten nicht direkt für den Anwender greifbar sind.

Aufgabe der vorliegenden Erfindung ist es, eine Vorrichtung zur Notfallversorgung eines Patienten derart zu konstruieren, daß eine optimierte Tragstruktur bereitgestellt wird.

Diese Aufgabe wird erfindungsgemäß dadurch gelöst, daß der Träger mindestens bereichsweise einen Abstand zu einer von der Rückseite aufgespannten Ebene aufweist.

Die im Folgenden beschriebenen Ausführungsformen der erfindungsgemäßen Idee können einzeln oder in Kombination miteinander verwendung finden.

Die Erfindung sieht vor, dass eine mobile Einheit als Träger für verschiedene Funktionseinheiten dem Helfer den Transport einer oder verschiedenster Funktionseinheiten zum Unfallort zu erleichtert und ihm unnötige, doppelte Wege erspart. Dies hat den Vorteil, dass der Helfer schneller und besser ausgestattet zum Unfallort oder zum Aufenthaltsort des Verletzten gelangen kann. Die Funktionseinheit kann ein Diagnosegerät oder ein Therapiegerät, beispielsweise ein Beatmungsgerät, ein Defibrillator, Pulsoximeter, Notfallgerät, Diagnosegerät, Absauggerät, ein Gerät zur Sauerstoffverabreichung, eine Schnittstelle oder ein sonstiges medizinisches Gerät sein.

Die erfindungsgemäße Idee sieht vor, dass eine Tragstruktur verschiedene im Blech vorgenommene Abkantungen hat, welche miteinander durch Schweißen, Nieten, Schrauben oder eine sonstige verbindungstechnik verbunden sind. Dazu kann ebenfalls ein Träger, welcher ebenfalls aus Blech gekantet worden sein kann, zählen sowie separat an der Tragstruktur angebracht sein. Daneben sind weitere Materialien und Formen als Ersatz für ein Blech vorstellbar und Bestandteil der erfindungsgemäßen Idee. Ferner ist die Ausführung der Tragstruktur und des Trägers aus Blech oder einem anderen metallischem Material angedacht.

In einer weiteren Ausführungsform kann die mobile Einheit auch teilweise aus einem nicht-metallischen Material hergestellt sein.

In einer Ausführungsform der Erfindung spannt die Tragstruktur, als zentrales Element, verschiedene Hüllflächen bzw. -ebenen durch ihre Begrenzungen auf. Eine Fläche kann die Rückseite sein. In einer bevorzugten Ausführungsform der Erfindung ist der Träger, welcher die Funktionseinheiten haltert, mindestens bereichsweise in einem Abstand zur Rückseite an die Tragstruktur angekoppelt.

Der Abstand kann in einer weiteren Ausführungsform der Erfindung auch zu einer andere Seite bzw. Ebene der Tragstruktur eingehalten werden. Auch kann die Form des Trägers und/oder eine Seite bzw. Ebene der Tragstruktur eben, wellig und/oder stufenförmig geformt sein. Als besonders vorteilhaft ist der Träger parallel zur Rückseite der Tragstruktur angeordnet.

Als eine weitere Ausführungsform kann der Träger separat und/oder einteilig mit der Rückseite verbunden sein. Ferner kann die Verbindung zwischen Träger und die Tragstruktur mindestens mit zwei Punkten durch eine Niet- und/oder Schraubverbindung gebildet werden.

Um einem erforderlichen Abstand herstellen zu können, ist in einer Ausführungsform daran gedacht, den Träger mit einer Seite der Tragstruktur über mindestens einen abgesetzten Fuß durch eine Niet- und/oder Schraubverbindung zu verbinden. Dabei kann der Fuß Teil des Trägers und/oder der Tragstruktur sein. Durch den Fuß kann der Abstand zwischen dem Träger und einer Seite und/oder der Rückseite vorgegeben werden.

In einer weiteren Ausführungsform der Erfindung kann ein bereichsweiser Abstand von Träger und Tragstruktur durch verstellbare Füsse eingestellt werden. Dazu können in die Füsse Bohrungen oder Schienen eingebracht sein, welche dann mit dem Gegenpart, Träger, Tragstruktur oder Fuss eines der beiden Teile, verbunden werden können.

Ein Aufnahmeraum, welcher durch die Tragstruktur, den Träger und/oder einem anderen mit der mobilen Vorrichtung in Verbindung stehenden Teil mindestens teilweise begrenzt werden kann, kann in einer Ausführungsform durch eine Öffnung erreichbar sein. Diese Öffnung kann durch eine Öffnung in der Tragstruktur, des Trägers oder von einer durch den Träger und die Tragstruktur geschaffene Öffnung gebildet werden. So ist es möglich, den Aufnahmeraum von außen zu erreichen. Besonders bevorzugt ist der Aufnahmeraum nach hinten, seitlich geöffnet und/oder geschlossen.

Der Aufnahmeraum ist erfindungsgemäß für die Aufnahme von beispielsweise Medikamenten, Begleitinformationen, Dokumentationsmappen einer Ladeschnittstelle, sonstigen Unterlagen und/oder einer Box mindestens teilweise auffüllbar.

Um auf die verschiedenen Arten von aufzunehmenden Teilen und deren Erreichbarkeit eingehen zu können, ist erfindungsgemäß vorgesehen, dass die Form der Öffnungen rund, rechteckig und/oder sonstig ausgeformt sein kann.

Auch können in einer besonders bevorzugten Ausführungsform mindestens zwei Öffnungen zueinander versetzt und/oder zueinander in gleicher Höhe in einem Abstand zu einer Kante der Tragstruktur angeordnet sein.

Ein weiterer Vorteil der Erfindung stellt die einfache Wartung und Montage der Funktionseinheiten auf der Tragstruktur und/oder auf dem Träger dar. Dabei kann durch eine in der Tragstruktur, dem Träger oder von einer Kombination dieser beiden Elemente gebildeten Öffnung die Wartung der Funktionseinheiten, einer Schnittstelle oder die Montage der Funktionseinheiten durchgeführt werden.

Um ein schnelles und einfaches Erreichen des Aufnahmeraumes und/oder um eine einfache Wartung durchführen zu können bzw. um an die benötigten Stellen einfach heranzukommen, ist in einer weiteren Ausführungsform daran gedacht, die Öffnungen mit Metall, Textil, Kunststoff oder einem anderen festen Material zu bedecken und/oder auszufüllen. Diese Teile können mit der mobilen Einheit in Verbindung stehen. Beispielswiese kann in einer bevorzugten Ausführungsform der Erfindung eine Box aus Metall aus Metall, Kunststoff und/oder Textil den Aufnahmeraum mindestens teilweise ausfüllen.

In einem Ausführungsbeispiel ist der Abstand zwischen Tragstruktur und Träger nicht größer als 33+/- 10 mm, in einem anderen nicht größer als 120 mm.

Um die Box oder den Aufnahmeraum vor einem unrechtmäßigen Öffnen und/oder den Inhalt der Box vor Sonneneinstrahlung oder sonstigen Einflüssen zu schützen, kann die Box und/oder der Aufnahmeraum eingeständig verschließbar sein. In einer weiteren Ausführungsform kann der Aufnahmeraum und/oder die Box mechanisch und lösbar mit der Tragstruktur verbunden werden. Beispielsweise kann der Aufnahmeraum und/oder die Box nur in Verbindung mit der Tragstruktur oder einer Öffnung geschlossen bzw. abgeschlossen werden. Das Abschließen kann durch ein Schloss geschehen, bei dem der Riegel hinter eine Seite der Tragstruktur und/oder des Trägers bewegt werden kann.

Ferner kann in einer weiteren Ausführungsform der Aufnahmeraum und/oder die Box durch eine formschlüssige Verbindung mit der Tragstruktur und/oder dem Träger wenigstens teilweise geschlossen werden.

Ein weiterer Vorteil der Erfindung ist Möglichkeit, die Funktionseinheiten ungeachtet einer Schnittstelle oder sonstiger bestehender Bestandteile auf einer Struktur anzuordnen, welche sich direkt auf der Rückseite des Trägers und/oder der Tragstruktur im Bereich von Tragstruktur und Träger befinden kann.

Um die gesamte mobile Einheit transportieren zu können, kann die Tragstruktur über einen Haltegriff, einen Tragegurt, Normschienenadapter, eine Tasche und/oder Bettenhalterung verfügen.

In den Zeichnungen der Erfindung sind Ausführungsbeispiele schematisch dargestellt. Es zeigen:
- Fig. 1:: Allgemeine Darstellung der mobilen Einheit
- Fig. 2:: Aufbau der Tragstruktur und eines Trägers
- Fig. 3:: Rückseitige Anordnung der mobilen Einheit
- Fig. 4:: Abbildung der Tragstruktur
- Fig. 5:: Box zur Aufbewahrung von verschiedenen Dingen, wie Medikamente, Begleitinformationen
- Fig. 6:: Eingelassene Box in die mobile Einheit

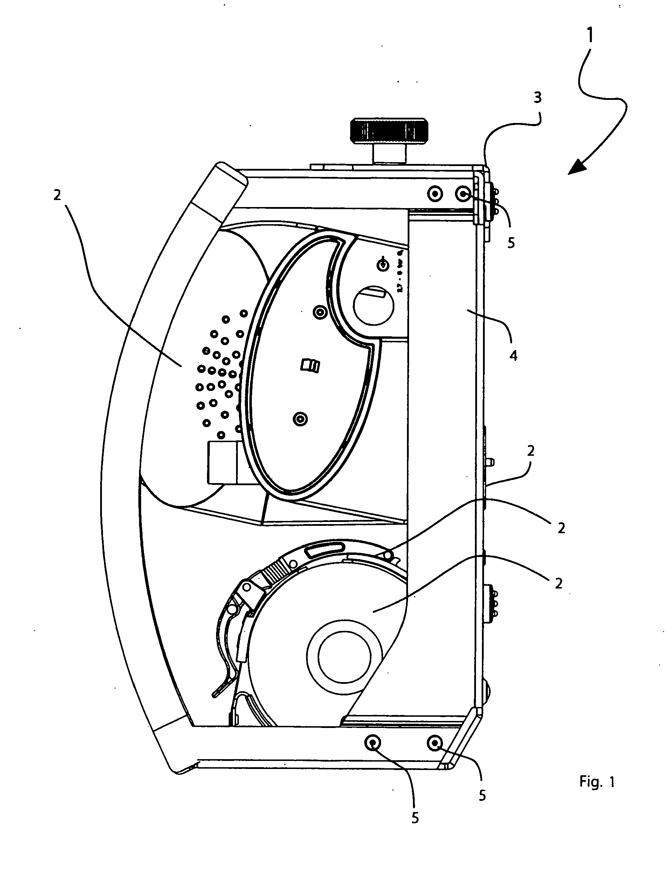

Fig. 1 zeigt eine allgemeine Anordnung der mobilen Einheit (1). Diese besteht aus verschiedensten Funktionseinheiten (2), hier Beatmungsgerät und Flaschenspannschellen für eine Gasdruckflasche, Halteklauen für die Anbringung der mobilen Einheit (1) an eine Krankenhausnormschiene oder sonstigen Mitteln, welchen die Möglichkeit des Einhängens bieten, und stehen allesamt mit der Tragstruktur (4) in Verbindung. Die Tragstruktur (4) selber ist aus einem oder mehreren Blechen gefertigt und gekantet. Zum Erreichen ihrer Stabilität sind überlappende Gebiete mit Nieten, Schrauben oder sonstigen Befestigungsmitteln (5) verbunden.

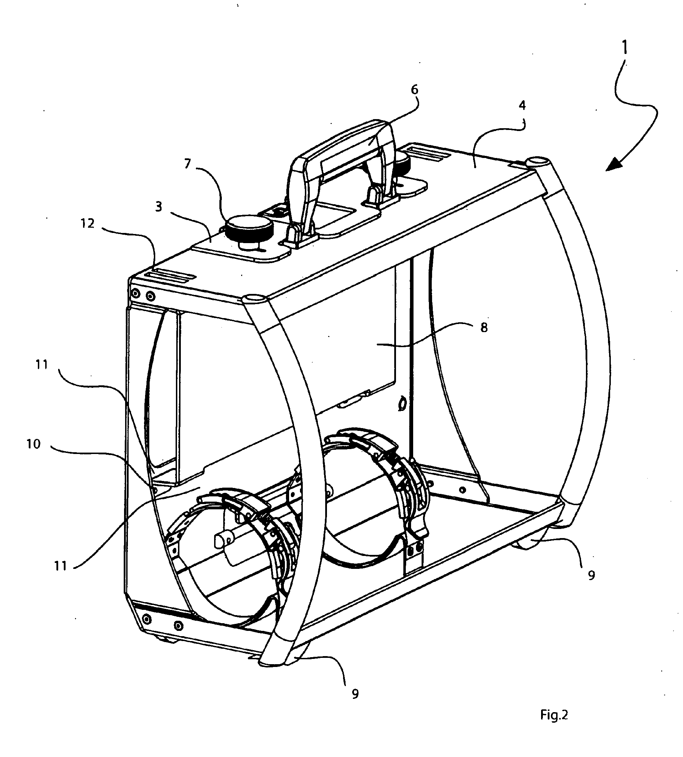

Fig. 2 zeigt den Aufbau einer Tragstruktur (4) und einen detaillierteren Blick auf den Träger (8), welcher zusammen mit der Tragstruktur (4) eine seitliche Öffnung (11) bildet. Des weiteren befinden sich an der Tragstruktur (4) Standfüße (9), sowie Rändelschrauben und Befestigungsmittel (7) zur Einstellung der Halteklaue/-schiene (3). Zum einfachen Transport der mobilen Einheit (1) ist an die Tragstruktur ein Haltegriff (6) angebracht. Ferner können in die Tragstruktur (4) Mittel zur Befestigung von Halteriemen (12) eingebracht werden. In die seitlichen Öffnungen können Begleitdokumente eingefügt werden, welche zur Bedienung von auf der mobilen Einheit (1) befestigten Funktionselementen (nicht gezeigt) dienen können.

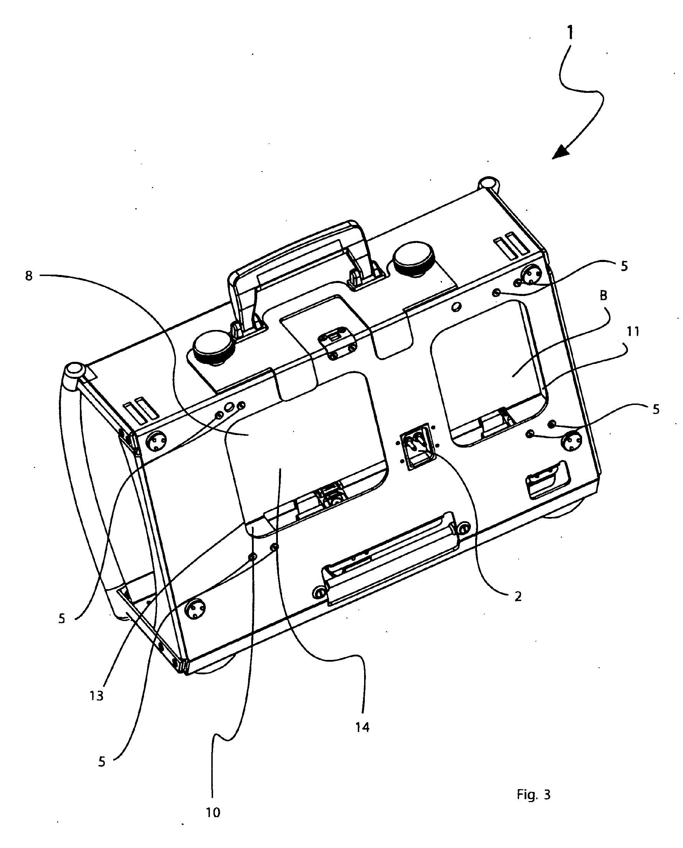

Fig. 3 zeigt die rückseitige Anordnung einer mobilen Einheit (1), insbesondere sind hier die beiden rückseitigen Öffnungen (13) zu nennen, welche im wesentlichen rechteckig geformt sind, aber auch in einer Form rund, als Ellipse als Kreis oder in einer sonstigen Kombination aus eckigen und/oder runden Abschnitten geformt sein können. Ferner ist hier ein weiteres Funktionselement (2), hier eine Schnittstelle zur Ladung und oder zum Austausch von Daten, gezeigt. Durch den Träger (8) und seine Füsse (10) kann mittels der Niete (5) ein Abstand zwischen Träger und Tragstruktur geschaffen werden. Beide Elemente, Träger(8) und Tragstruktur (4), schaffen einen Aufnahmeraum (14). Der Aufnahmeraum (14) kann mit Begleitdokumenten, Defibrillationselektroden (nicht gezeigt) mindestens teilweise ausgefüllt werden.

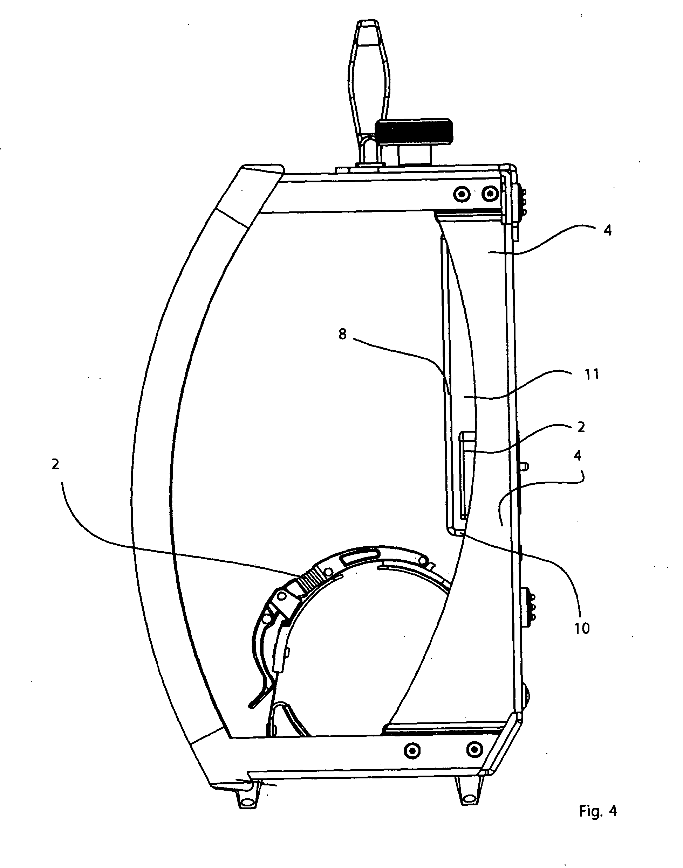

Fig. 4 zeigt eine weitere Abbildung der Tragstruktur (4), an der ein Träger (8) über Füsse (10), und eine Funktionseinheit (2), hier Schnittstelle, befestigt sind.

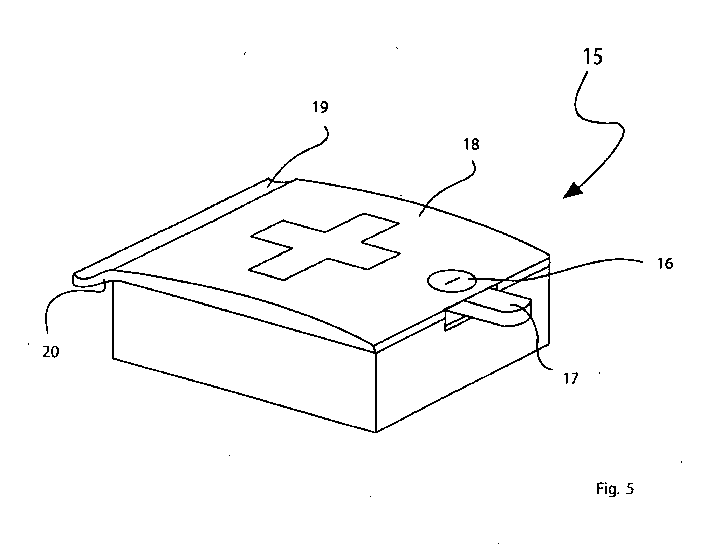

Fig 5. zeigt den Aufbau einer festen Box (15) aus Metall. Diese kann über einen Deckel (18) und ein Schloss (16) mit einem Schließbolzen (17) verfügen. Diese Box (15) kann ebenfalls Begleitinformationen, wichtige Dokumente und Medikamente aufnehmen, ähnlich eine Geldkassette. Die Box kann entweder eine abschließbare Einheit bilden oder in Kombination mit der Tragstruktur respektive dem Träger eine formschlüssige, nur durch Öffnen des Schlosses lösbare Verbindung eingehen. Über die Scharnierachse kann der Deckel (18) geöffnet werden. Mit Hilfe des abgesetzten Deckelabschnittes kann die Box in den Aufnahmeraum der mobilen Einheit eingeführt werden.

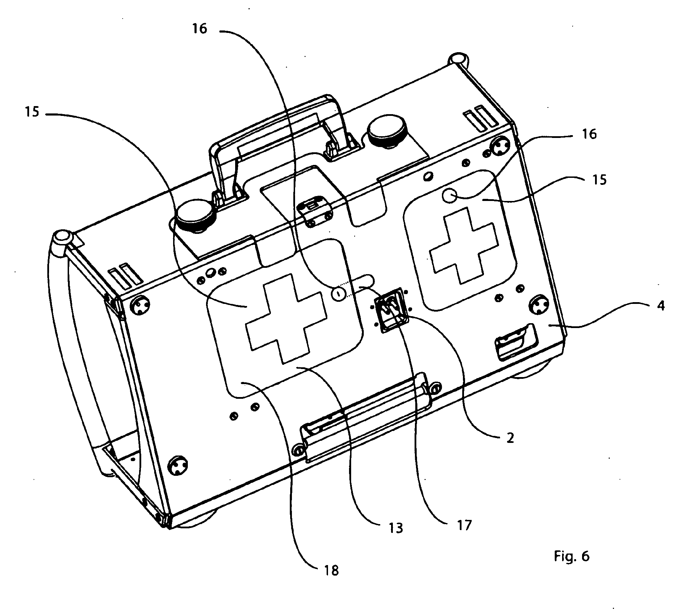

Fig. 6 zeigt die in den Aufnahmeraum eingefügte Box (15) zur Aufnahme von beispielsweise Medikamenten. Der Deckel (18) passt sich der Form der rückseitigen Öffnung (13) in der Tragstruktur (4) an, so dass idealerweise eine bündige Anordnung einer Seite der Tragstruktur (4) und der Oberfläche des Deckels (18) hergestellt wird. Verschlossen und mit der Tragstruktur lösbar verbunden wird die Box (15) mit dem Schloss (16) und dem Schließbolzen (17), welcher unter die Rückseite der Tragstruktur greift. Die Box (15) respektive deren Bewegungsfreiheit von der Tragstruktur weg, wird vom Träger (8) aus der entegegengesetzten Richtung begrenzt.

## Patentansprüche

1. Vorrichtung zur Notfallversorgung eines Patienten, die als eine mobile Einheit ausgebildet ist, die eine Tragstruktur mit einer Rückseite sowie mindestens einer Funktionseinheit aufweist und bei der die Funktionseinheit durch einen Träger gehaltert ist, **dadurch gekennzeichnet, dass** der Träger mindestens bereichsweise einen Abstand zu einer von der Rückseite aufgespannten Ebene aufweist.

2. Vorrichtung nach Anspruch 1, **dadurch gekennzeichnet, dass** der Träger parallel und/oder schräg zur Rückseite angeordnet ist.

3. vorrichtung nach einem der Ansprüche 1 oder 2, **dadurch gekennzeichnet, dass** der Träger separat und/oder einteilig mit der Rückseite verbunden ist.

4. Vorrichtung nach einem der Ansprüche 1 bis 3, **dadurch gekennzeichnet, dass** durch den Träger und mindestens eine Seite der Tragstruktur ein Aufnahmeraum in mindestens einer Dimension begrenzt ist.

5. Vorrichtung nach einem der Ansprüche 1 bis 4, **dadurch gekennzeichnet, dass** der Aufnahmeraum über zumindest eine Öffnung von außen erreichbar ist.

6. Vorrichtung nach einem der Ansprüche 1 bis 5, **dadurch gekennzeichnet, dass** die Tragstruktur und/oder der Träger mindestens eine Öffnung bilden.

7. Vorrichtung nach einem der Ansprüche 1 bis 6, **dadurch gekennzeichnet, dass** mindestens zwei Öffnungen zueinander versetzt und/oder zueinander in gleicher Höhe und in einem Abstand zu einer Kante der Tragstruktur angeordnet sind.

8. Vorrichtung nach einem der Ansprüche 1 bis 7, **dadurch gekennzeichnet, dass** der Aufnahmeraum durch die Aufnahme von beispielsweise Medikamenten, Begleitinformationen, Dokumentationsmappen einer Ladeschnittstelle, Defibrillationselektroden, sonstigen Unterlagen und/oder einer Box mindestens teilweise ausgefüllt ist.

9. Vorrichtung nach einem der Ansprüche 1 bis 8, **dadurch gekennzeichnet, dass** der Träger mit einer Seite der Tragstruktur über mindestens einen abgesetzten Fuß durch eine Niet- und/oder Schraubverbindung verbunden ist.

10. Vorrichtung nach einem der Ansprüche 1 bis 9, **dadurch gekennzeichnet, dass** durch den Fuß der Abstand zwischen Träger und einer Seite und/oder die Rückseite vorgegeben ist.

11. Vorrichtung nach einem der Ansprüche 1 bis 10, **dadurch gekennzeichnet, dass** die Öffnungen durch Metall, Textil, Kunststoff oder einem anderen festen Material bedeckbar/ausfüllbar sind.

12. Vorrichtung nach einem der Ansprüche 1 bis 11, **dadurch gekennzeichnet, dass** der Träger den Aufnahmeraum begrenzt.

13. Vorrichtung nach einem der Ansprüche 1 bis 12, **dadurch gekennzeichnet, dass** die Box und/oder der Aufnahmeraum eigenständig verschließbar ist.

14. Vorrichtung nach einem der Ansprüche 1 bis 13, **dadurch gekennzeichnet, dass** der Aufnahmeraum und/oder die Box mechanisch und lösbar mit der Tragstruktur verbindbar ist.

15. Vorrichtung nach einem der Ansprüche 1 bis 14, **dadurch gekennzeichnet, dass** der Aufnahmeraum und/oder die Box durch eine formschlüssige Verbindung mit der Tragstruktur und/oder dem Träger gehaltert ist.

16. Vorrichtung nach einem der Ansprüche 1 bis 15, **dadurch gekennzeichnet, dass** im Bereich von Tragstruktur und Träger eine Schnittstelle angebracht ist, die die Anordnung der Funktionseinheiten auf dem Träger nicht einschränkt.
